(19) Europäisches Patentamt
European Patent Office
Office européen des brevets

(11) Publication number: **0 201 633 A2**

# (12) EUROPEAN PATENT APPLICATION

(21) Application number: 85116062.2

(22) Date of filing: 17.12.85

(51) Int. Cl.[4]: **C 07 D 493/10**
C 07 K 13/00, C 07 K 15/00
G 01 N 33/531, G 01 N 33/94
G 01 N 21/64
//(C07D493/10, 311:00, 307:00)

(30) Priority: 20.12.84 US 684770

(43) Date of publication of application: 20.11.86 Bulletin 86/47

(84) Designated Contracting States: BE CH DE FR GB IT LI NL SE

(71) Applicant: ABBOTT LABORATORIES
14th Street and Sheridan Road North St
North Chicago Illinois 60064(US)

(72) Inventor: Ungemach, Frank Schneider
1221 S. Main St.Apt.2B
Antioch, IL 60002(US)

(72) Inventor: Nystrom, David Donald
4350 Finch Court, Apt.8
Gurnee, IL 60031(US)

(72) Inventor: Keegan, Candace Linda
671 Cardinal Court
Grayslake, IL 60030(US)

(72) Inventor: Stroupe, Stephen Denham
606 Roosevelt Drive
Libertyville, IL 60048(US)

(74) Representative: Modiano, Guido et al,
MODIANO, JOSIF, PISANTY & STAUB Modiano & Associati Via Meravigli, 16
I-20123 Milan(IT)

## (54) Substituted anilide compounds and their use.

(57) The present invention is directed to a fluorescent polarization assay for acetaminophen, to the various components needed for preparing and carrying out such an assay, and to methods of making these components. Specifically, tracers, immunogens and antibodies are disclosed, as well as methods for making them. The tracers and the immunogens are made from substituted anilide compounds. A fluorescein moiety is included in the tracer, while a poly-(amino acid) forms a part of the immunogen. The antibodies are prepared in response to the immunogen. The assay is conducted by measuring the degree of polarization retention of plane polarized light that has been passed through a sample containing antiserum and tracer.

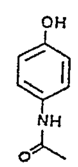

Fig. 1

EP 0 201 633 A2

Croydon Printing Company Ltd.

TITLE MODIFIED
see front page



# ACETAMINOPHEN ASSAY, TRACERS, IMMUNOGENS AND ANTIBODIES

## BACKGROUND OF THE INVENTION

### 1. Technical Field

The present invention relates to a method and reagents for a fluorescence polarization immunoassay procedure for determining the amount of acetaminophen in fluids, especially biological fluids such as serum, plasma or urine, and to a method of making the reagents. More specifically, the invention relates to (1) reagents (tracers and antibodies) for determining the amount of acetaminophen in a sample; (2) immunogen compounds used to raise antibodies; (3) synthetic methods (for making the tracer and immunogen compounds); and (4) analytical methods for conducting the assay.

### 2. Background Art

Acetaminophen is a common aspirin substitute often prescribed as an analgesic and antipyretic and is generally considered to be relatively safe. However, high doses can cause liver damage, and the availability of over-the-counter preparations can lead to accidental or intentional overdoses. Moreover, liver damage caused by excessive acetaminophen is often not symptomatic until

several days after the overdose, by which time it may be too late to effectively administer an antidote for acetaminophen poisoning. Accordingly, in diagnosing cases of possible overdose it is important to monitor the drug half-life because this is an indication of the extents of toxicosis. Acetaminophen can be determined in urine,blood serum or plasma.

In the past, patient serum or plasma acetaminophen levels have typically been measured by colorimetric thin layer chromatography, enzyme immunoassay, or high performance liquid chromatography (HPLC) assays.
These methods are not without drawbacks; for example, the assay time can typically be lengthy.

In assays for other substances, competitive binding immunoassays have provided a more satisfactory alternative. Typically, competitive binding immunoassays are used for measuring ligands in a test sample. (For the purposes of this disclosure, a "ligand" is a substance of biological interest to be quantitatively determined by a competitive binding immunoassay technique.) The ligands compete with a labeled reagent, or "ligand analog," or "tracer," for a limited number of receptor binding sites on antibodies specific to the ligand and ligand analog. The concentration of ligand in the sample determines the amount of ligand analog which binds to the antibody: the amount of ligand analog that will bind is inversely proportional to the concentration of ligand in the sample, because the ligand and the ligand analog each bind to the antibody in proportion to their respective concentrations.

Fluorescence polarization provides a quantitative means for measuring the amount of tracer-antibody conjugate produced in a competitive binding immunoassay. Fluorescence polarization techniques are based on the principle that a fluorescent labeled compound, when excited by plane polarized light, will emit fluorescence having a degree of polarization inversely related to its rate of rotation. Accordingly, when a

tracer-antibody conjugate having a fluorescent label is excited with plane polarized light, the emitted light remains highly polarized because the fluorophore is constrained from rotating between the time that light is absorbed and emitted. In contrast, when an unbound tracer is excited by plane polarized light, its rotation is much faster than the corresponding tracer-antibody conjugate and the molecules are more randomly oriented. As a result, the light emitted from the unbound tracer molecules is depolarized.

Such fluorescence polarization techniques have been applied in U.S. Patent No. 4,420,568 to Wang, et al., which is directed to the use of a triazinylaminofluorescein moiety as the fluorophore. The present invention offers an advance in the art beyond the Wang, et al. patent, in that highly sensitive tracers, a method for making the tracers, and an assay using the tracers are provided specifically for the determination of acetaminophen. The assay conducted in accordance with the present invention is particularly accurate, as will be explained below.

SUMMARY OF THE INVENTION

The present invention is directed to a fluorescence polarization assay for acetaminophen; to tracers, immunogens and antibodies for use in the assay; and to methods for making the tracers, immunogens and antibodies.

A first aspect of the invention relates to the discovery of unique tracers and immunogens having novel structures. According to the first aspect of the invention, the tracers and the immunogens can both be represented by the structural formula shown in Figure 5 where:

Q is a poly(amino acid), a poly(amino acid) derivative, fluorescein, or a fluorescein derivative;

X is NH or CO;

n is 0, 1 or 2;

R is a linking group including up to 2 heteroatoms when Q is a poly(amino acid) or a poly(amino acid) derivative and up to 4 heteroatoms when Q is fluorescein or a fluorescein derivative, and having a total of from 0 to 8 carbon atoms and heteroatoms;

W is O or S when Q is fluorescein or a fluorescein derivative; and W is O when Q is a poly(amino acid) or poly(amino acid) derivative;

Y is OH, $NH_2$, $CH_3$, F, Cl, Br or H when Q is fluorescein or a fluorescein derivative, and Y is OH, $NH_2$, $CH_3$, F or Cl when Q is a poly(amino acid) or a poly(amino acid) derivative; and

$Z_1$, $Z_2$, $Z_3$ and $Z_4$ are each independently H or F when Q is fluorescein or a fluorescein derivative, and are each H when Q is a poly(amino acid) or a poly(amino acid) derivative.

When Q is a poly(amino acid) or a derivative thereof, the compound can be used as an immunogen. When Q is a fluorescein or a derivative thereof, the compound can be used as a tracer.

A second aspect of the invention relates to antibodies raised by the novel immunogen. According to the second aspect of the invention, antibodies are prepared in response to a compound according to Claim 1 when Q is a poly(amino acid) or a derivative thereof.

According to a third aspect of the invention, an immunogen is made by a method comprising the step of coupling a compound represented by the structural formula shown in Figure 2, where:

X is $NH_2$, COOH, CN, CHO, Br, I or OH;

R is a linking group including up to 2 heteroatoms and having a total of from 0 to 8 carbon atoms and heteroatoms when X is COOH, and having a total of from 1 to 8 carbon atoms and heteroatoms when X is $NH_2$, CN, CHO, Br, I or OH; and

Y is OH, $NH_2$, $CH_3$, F or Cl;

with a poly(amino acid) or a derivative of a poly(amino acid).

According to a fourth aspect of the invention, a method is provided for making a tracer by coupling a compound represented by the structural formula shown in Figure 3, where:

X is $NH_2$, COOH, CN or OH;

R is a linking group including up to 4 heteroatoms and having a total of from 0 to 8 carbon atoms and heteroatoms when X is COOH, and having a total of from 1 to 8 carbon atoms when X is $NH_2$, CN or OH;

U is H or the structure $\overset{W}{\overset{\|}{C}}$-R-X where W is O or S;

Y is OH, $NH_2$, $CH_3$, F, Cl, Br or H; and

$Z_1$, $Z_2$, $Z_3$ and $Z_4$ are each independently H or F;

with fluorescein or a derivative of fluorescein.

According to a fifth aspect of the invention, a process for measuring concentration of acetaminophen is provided. A sample is contacted with acetaminophen antiserum, and a fluorescein containing acetaminophen derivative capable of producing a detectable fluorescence polarization response to the presence of the acetaminophen antiserum. Plane polarized light is then passed through the solution to obtain a fluorescence polarization response, and this response is detected as a measure of the amount of acetaminophen in the sample.

Further objects and attendant advantages of the invention will be best understood from a reading of the following detailed description taken together with the drawings and the Examples.

BRIEF DESCRIPTION OF THE DRAWINGS

In the following Figures the symbol "Fl" represents a fluorescein moiety, and the various other symbols are noted in the Detailed Description.

Figure 1 shows the general structure of the class of acetaminophen to be quantitatively determined in accordance with the present invention.

Figure 2 shows a class of reactants for a method of making an immunogen in accordance with the present invention.

Figure 3 shows a class of reactants for a method of making a tracer in accordance with the present invention.

Figure 4 shows the alternate structural formulae and names of the fluorescein moiety included in the tracers of the present invention.

Figure 5 shows a general structural formula for the tracers and the immunogens of the present invention.

Figure 6 shows a general structural formula for the immunogens of the present invention.

Figure 7 shows a general structural formula for the tracers of the present invention.

Figure 8 shows a structural formula for preferred immunogens of the present invention.

Figure 9 shows a structural formula for preferred tracers of the present invention.

Figure 10 shows a precursor for the immunogens shown in Figures 6 and 8 and for the tracers shown in Figures 7 and 9.

Figure 11 shows various linkages that couple the fluorescein moiety to the precursor at the X position in Figure 10, when Figure 10 represents a precursor for the tracers shown in Figures 7 and 9.

Figures 12 through 16 show various examples of structures of tracers in accordance with the present invention.

Figures 17 through 20 show various examples of structures of hapten reactants used to form the immunogens of the present invention.

## DETAILED DESCRIPTION OF THE PREFERRED EMBODIMENTS

The various aspects of the invention will now be discussed in relation to the Figures and/or the Examples.

The present invention involves the use of fluorescein and derivatives of fluorescein. In particular, a necessary property of fluorescein and its derivatives for the usefulness of the tracer compounds is the fluorescence of fluorescein. Fluorescein exists in two tautomeric forms, illustrated in Figure 4, depending on the acid concentration (pH) of the environment. In the open (acid) form, there are a number of conjugated double bonds which make that form of fluorescein (and compounds containing a fluorescein moiety) capable of absorbing blue light and emitting green fluorescence after an excited state lifetime of about 4 nanoseconds. When the open and closed forms coexist,

the relative concentration of molecules in the open and closed forms is easily altered by adjustment of the pH level. Generally, the tracer compounds of the present invention exist in solution as biologically acceptable salts such as sodium, potassium, ammonium and the like, which allows the compounds to exist in the open, fluorescent form, when employed in the analytical methods of the present invention. The specific salt present will depend on the buffer employed to adjust the pH level. For example, in the presence of a sodium phosphate buffer, the compounds of the present invention will generally exist in the open form, as a sodium salt.

As used herein, the term "fluorescein," either as an individual compound or as a component of a larger compound, is meant to include both the open and closed forms, if they exist for a particular molecule, except in the context of fluorescence. An open form is necessary for the fluorescence to occur.

The numbering of carbon atoms of the fluorescein molecule varies, depending upon whether the open or closed form of the molecule is considered. Accordingly, the literature concerning fluorescein and its compounds is not uniform as to carbon atom numbering. In the closed form, the para-carbon to the carbonyl of the lactone on the phenyl ring is numbered 6 (this is sometimes denominated "isomer II"). In the open form, the para-carbon to the carboxylic acid group on the phenyl ring is numbered 5 (this is sometimes denominated "isomer I"). Figure 4 illustrates these isomers. For the purpose of this disclosure the numbering of the closed form is adopted because the raw materials used in the syntheses are most popularly numbered with that system. The carbon atom of fluorescein and its compounds which is opposite the carboxyl group is therefore numbered "6" for the purposes of the present disclosure.

A tracer which is not complexed to an antibody is free to rotate in less than the time required for absorption and re-emission of fluorescent light. As a result, the re-emitted light is relatively randomly oriented so that the fluorescence polarization of a tracer not complexed to an antibody is low, approaching zero. Upon complexing with a specific antibody, the tracer-antibody complex thus formed assumes the rotation of the antibody molecule which is slower than that of the relatively small tracer molecule, thereby increasing the polarization observed. Therefore, when a ligand competes with the tracer for antibody sites, the observed polarization of fluorescence of the tracer-antibody complex becomes a value somewhere between that of the tracer and tracer-antibody complex. If a sample contains a high concentration of the ligand, the observed polarization value is closer to that of the free tracer, i.e., low. If the test sample contains a low concentration of the ligand, the polarization value is closer to that of the bound tracer, i.e., high. By sequentially exciting the reaction mixture of an immunoassay with vertically and then horizontally polarized light and analyzing only the vertical component of the emitted light, the polarization of fluorescence in the reaction mixture may be accurately determined. The precise relationship between polarization and concentration of the ligand to be determined is established by measuring the polarization values of calibrators with known concentrations. The concentration of the ligand can be extrapolated from a standard curve prepared in this manner.

The particular tracers formed in accordance with this invention have been found to produce surprisingly good assays, as will be demonstrated later in this disclosure.

1. The Reagents

Both the immunogens and the tracers of the present invention can be represented by the general structural formula set forth in the Summary of the Invention, and illustrated in Figure 5. When Q is a poly(amino acid), the structure represents the immunogen; when Q is a fluorescein moiety, the structure represents the tracer.

The objective is to have competition between acetaminophen and the tracer for the recognition sites of the antibody. Great variations in the structure of the haptens and tracers are allowed in achieving this goal. For the purposes of this invention, "haptens" are precursors of the immunogens, comprising generally a substituted anilide derivative and a linking group to the poly(amino acid) carrier.

a. The Structure of the Immunogens

Usable antibodies can be produced from a variety of anilide derivatives. Immunogens made from compounds that have OH, $NH_2$, $CH_3$, F or Cl in place of the -OH on acetaminophen can produce antibodies in animals; such antibodies are useful in an acetaminophen assay according to the invention when combined with the appropriate tracer.

The immunogens of the present invention have the general structural formula shown in Figure 6, and in the preferred form of the invention, the immunogens have the structural formula shown in Figure 8. This structure is preferred because the best recognition of the OH group and aromatic ring occurs when the ring is substituted at a position as distant as possible from these groups. Although bovine serum albumin is the poly(amino acid) in this preferred form, it should be understood that various protein carriers may be

employed, including albumins, serum proteins, e.g., globulins, ocular lens proteins, lipoproteins and the like. Illustrative protein carriers include bovine serum albumin, keyhole limpet hemocyanin, egg ovalbumin, bovine gamma-globulin, thyroxine binding globulin, etc. Alternatively, synthetic poly(amino acids) may be prepared having a sufficient number of available amino groups such as lysines.

The immunogens can be prepared by coupling a compound of the class shown in Figure 2 with a poly(amino acid) or a derivative of a poly(amino acid), as will be discussed in the context of the synthetic method and the Examples below.

b. The Structure of the Tracers

The possible variations in the structure of the tracers of the invention are even greater than the possible variations in the structure of the haptens thereof. The tracers of the present invention have the general structural formula shown in Figure 7, where Fl represents a fluorescein moiety or a fluorescein derivative. In a preferred form of the invention, the tracers have the structural formula shown in Figure 9.

The tracer is an anilide derivative that is linked to a fluorescein derivative by, for example, an amido, amidino, triazinylamino, carbamido, thiocarbamido, carbamoyl, thiocarbamoyl, or sulfonylcarbamoyl group, as shown in Figure 11. The tracers are prepared by linking the appropriate fluorescein derivative to an anilide derivative containing an amino, carboxylic acid, hydroxy, imidate, hydrazide, isocyanate, thioisocyanate, chloroformate, chlorothioformate, chlorosulfonylcarbamoyl, or the like group, as will be discussed in the context of the synthetic method and the Examples below.

By way of example, any of the following fluorescein derivatives can be used:

$Fl-NH_2$ fluorescein amine

$Fl-CO_2H$ carboxyfluorescein

$Fl-NHCOCH_2I$ α-iodoacetamidofluorescein

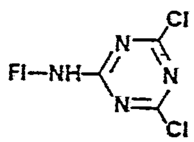

(DTAF) 2,4-dichloro-1,3,5,-triazin-2-yl amino-fluorescein

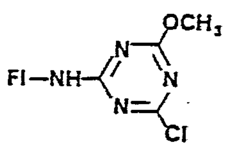

4-chloro-6-methoxy-1,3,5-triazin-2-ylamino fluorescein

Fl-NCS fluorescein thioisocyanate

2. <u>The Antibodies</u>

The antibodies of the present invention are prepared by developing a response in animals to the immunogens described above. The immunogen is administered to animals such as rabbits or sheep by a series of injections, in a manner well-known to those skilled in the art.

3. <u>Synthetic Methods</u>

Both the immunogens and the tracers of the present invention can be made from a precursor having the general structural formula shown in Figure 10, where

X is $NH_2$, COOH, CHO, CN, Br, I or OH when the preparation is directed to an immunogen, and $NH_2$, COOH, CN or OH when the preparation is directed to a tracer;

U is H, or the structure $\overset{W}{\overset{\|}{C}}-R-X$ where: W is O or S when the preparation is directed to a tracer and W is O where the preparation is directed to an immunogen; and R is a linking group including a total of from 1 to 8 carbon atoms and heteroatoms when X is $NH_2$, CN, CHO, Br, I or OH and a total of from 0 to 8 carbon atoms and heteroatoms;

Y is OH, $NH_2$, $CH_3$, F, Cl, Br or H when the preparation is directed to a tracer, and Y is OH, $NH_2$, $CH_3$ F or Cl when the preparation is directed to an immunogen; and

$Z_1$, $Z_2$, $Z_3$ and $Z_4$ are each independently H or F when the preparation is directed to a tracer, and are each H when the preparation is directed to an immunogen.

a. The Synthesis of the Immunogens

The immunogens of the present invention are made by coupling a hapten, such as that shown by the general structure of Figure 2 when X is $NH_2$, COOH, CN, CHO, bromine, iodine or OH, to a poly(amino acid). The poly(amino acid) moiety can be linked to the hapten by an amide, an amidine, an alkyl, a urea, a thiourea, a carbamate, or a thiocarbamate linkage. In a preferred embodiment, the poly(aminoacid) is bovine serum albumin (BSA), and the hapten is shown in Figure 18. The hapten is preferably coupled under conditions normally used to form alkyl linkages and such conditions are well known to those skilled in the art. It is most preferred that high pH conditions be used for forming the desired alkyl linkages as these are the most effective for forming these -- linkages in this context.

The immunogens are prepared by coupling a hapten that contains an $-NH_2$, $-CO_2H$, $-CONHNH_2$, -CNOR, -CHO, -Br, -I, -NCO, -NCS, -OCOCl or -OCSCl group to a poly(amino acid). The $-NH_2$ case can be coupled by activating the carboxylic acid group on the poly(amino acid) in the presence of the $-NH_2$ group. The activation of the carboxylic acid groups on the poly(amino acid) can be accomplished by mixing the hapten and the poly(amino acid) with 1-ethyl-3-(3-dimethylamino-propyl)carbodiimide (EDC), N,N'-dicyclohexylcarbodiimide (DCC), 1-cyclohexyl-3-(2-morpholinoethyl)carbodiimide metho -p-toluenesulfonate, or the like. The $-CO_2H$ case is also coupled by the activation method (EDC) or the active ester method, as described below in the tracer synthesis section. The $-CONHNH_2$ case is coupled in the same manner as for the non-aromatic amino case. The -CNOR case is coupled directly to the poly(amino acid). The -CHO case is coupled to the poly(amino acid) by reductive amination. The poly(amino acid) is mixed with the -CHO hapten and the resulting imine is reduced with sodium cyanoborohydride to yield alkylated amines on the poly(amino acid). The -Br and -I cases also produce alkylated amines on the poly(amino acid), but by direct coupling of the alkyl halide to the amine on the poly(amino acid). The isocyanate (-NCO), isothiocyanate (-NSC), chloroformate (-OCOCl) and chlorothioformate (-OCSCl) cases produce urea, thiourea, carbamate and thiocarbamate linkages, respectively. This is accomplished by direct coupling of the hapten to the poly(amino acid).

The synthesis of the above haptens (immunogen precursors where Y=OH, $NH_2$, $CH_3$, F or Cl) are accomplished in very similar ways. Figure 2 shows an immunogen precursor class in accordance with a preferred embodiment of the method of the present invention.

0201633

In general, the hapten is prepared by reaction of the appropriate aniline derivative, such as 4-hydroxyaniline, 4-fluoroaniline or the like, with the corresponding carboxylic acid derivative, X-R-$CO_2$H. This coupling can be accomplished by formation of the corresponding acid chloride, acid anhydride, active ester or mixed anhydride of the carboxylic acid derivative.

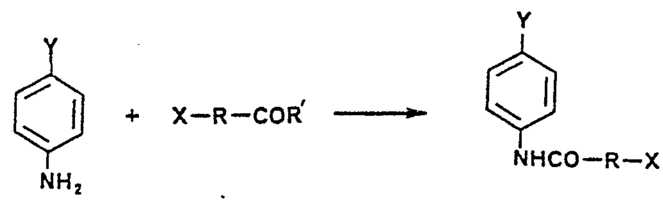

In the case where Y=$NH_2$, the starting material that will produce the best results is 4-nitroaniline (Y=$NO_2$). At the appropriate steps in the synthesis, the nitro group (Y=$NO_2$) is reduced to the amino group (Y=$NH_2$) by standard methods. In the cases where X=$NH_2$ and CHO, it is best to protect these groups, such as with a tert-butoxycarbamyl group or the like and by conversion to an acetal derivative, respectively. After the coupling is complete, the protecting groups are removed. In the case where X=$CO_2$H, it is best to utilize the corresponding cyclic anhydride, which is condensed with the appropriate aniline derivative to produce the hapten. When this is not readily possible or desirable,

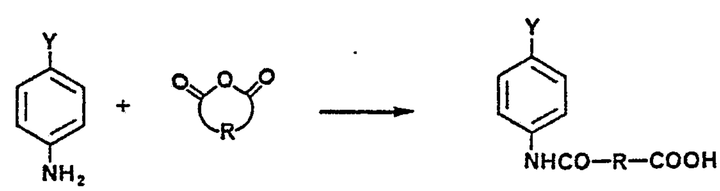

the X=$CO_2$H group can be protected as an ester, which can be easily hydrolized to the carboxylic acid group by standard methods.

The $X=NH_2$, $-CO_2H$, -Br, and -I derivatives are suitable to be coupled to produce immunogens. The nitrile derivatives (X=CN) are converted to alkoxy imidates (X=-CNOR) by treating the nitrile with anhydrous alcohol and hydrogen chloride gas. The alkoxy imidates are then ready for coupling. The hydrazide derivatives ($X=-CONHNH_2$) are prepared from the corresponding carboxylic acid derivatives by active ester coupling with hydrazine or by reacting hydrazine with the corresponding carboxylic ester derivative. The aldehyde derivatives (X=-CHO) are also suitable to be coupled to produce immunogens as described earlier. With the appropriate protection of the Y=OH or $NH_2$ groups, the amine ($X=NH_2$) is convertible to the isocyanate or thioisocyanate derivatives and the alcohol (X=OH) is convertible to the chloroformate and chlorothioformate derivatives by reaction of the amine or the alcohol with phosgene or thiophosgene. These derivatives are then coupled to a poly(amino acid) and the protecting groups are removed.

The iodo derivative can be prepared from the corresponding bromo or chloro derivatives by halogen exchange with sodium iodide or the like.

The aldehydes can be condensed with (aminohydroxy)alkylcarboxylic acids, such as $NH_2OCH_2CO_2H$, to produce substituted oxime derivatives. These are ready to be coupled to a poly(amino acid). The oxime alkyl carboxylic acid derivatives can be partially reduced to the corresponding (aminohydroxy)alkylcarboxylic acid derivatives, which also are ready to be coupled to a poly(amino acid). The same type of condensation and reduction can be accomplished with hydrazine and hydrazine derivatives.

<u>b. The Synthesis of the Tracers</u>

The tracers of the present invention are made by coupling a fluorescein moiety, or a derivative of

fluorescein, to the general structure shown in Figure 10 when X is $NH_2$, COOH, CNOR or OH. The fluorescein moiety can be linked to the amino, carboxyl, imidate or alkoxy functional group by an amide, an amidine, a urea, a thiourea, a carbamate, a thiocarbamate, triazinylamino or sulfonylcarbamate linkage, as shown in Figure 11. In the presently preferred embodiment, the fluorescein derivative is 6-carboxyfluorescein and this is coupled to a precursor shown in Figure 10. 6-carboxyfluorescein is coupled to 4-aminophenol by first forming the active ester of 6-carboxyfluorescein.

The active ester can be prepared from N-hydroxysuccinimide, 1-hydroxybenzotriazole, or p-nitrophenol via N,N'-dicyclohexylcarbodiimide activation in dry pyridine. Other solvents, such as dimethylformamide and dimethylsulfoxide, can be used. The structure is shown in Figure 17. Usable tracers can be prepared from a variety of anilide derivatives.

All anilide derivatives that have a terminal amino group, such as amino, hydrazinyl, hydrazido or the like, are coupled to carboxyfluorescein by the active ester method or the mixed anhydride method, and coupled to fluorescein isothiocyanate, DTAF or alkoxy DTAF by simply mixing the two materials in solution. The amino group can be converted to the isocyanate and thioisocyanate groups by reaction with phosgene and thiophosgene, respectively. These are then condensed with aminofluorescein to produce the tracer. The Y=OH and $NH_2$ groups require a protecting group for the conversion of the amino group to the isocyanate and the thioisocyanate groups. The protecting group can be removed after the coupling to fluorescein amine is complete.

All anilide derivatives that have a terminal carboxylic acid group, such as carboxylic acid, (amino-hydroxy)alkylcarboxylic acid or the like, are coupled to aminofluorescein by the active ester method. It is preferred that the $Y=NH_2$ group be protected during the active ester coupling.

All anilide derivatives that have a terminal hydroxy group can be coupled to fluorescein by reaction with DTAF, α-iodoacetamidofluorescein or fluorescein isothiocyanate in solution. The hydroxy group can be converted to the chlorosulfonylcarbamoyl, chloroformate and chlorothioformate groups by reaction with chlorosulfonylisocyanate, phosgene and thiophosgene, respectively. These derivatives are then coupled to aminofluorescein in solution to produce the tracer. The Y=OH and $NH_2$ groups require a protecting group for the coupling reactions and the conversion of the hydroxy group to the chlorosulfonylcarbamoyl, chloroformate and chlorothioformate groups. The protecting groups are removed after the coupling to the fluorescein derivatives.

All anilide derivatives that have a terminal nitrile group are converted to imidates in anhydrous alcohol in the presence of hydrogen chloride gas. The imidate is then coupled to fluorescein amine in solution to prepare the tracer.

The preparation of the various amino, carboxylic acid, hydroxy and nitrile derivatives of the anilide derivatives, with the exception of Y=Br,H and $Z_1$, $Z_2$, $Z_3$ and/or $Z_4$=F, were described above in the immunogen preparation section. The derivatives where Y=Br or H are prepared by the same method as Y=Cl. The derivatives where $Z_1$, $Z_2$, $Z_3$ and/or $Z_4$=F are prepared from the corresponding amino derivatives. The amino derivatives are prepared by reduction of the corresponding

nitro derivatives. The nitro derivatives are prepared from known benzene derivatives by known methods.

4. The Assay

The particular tracers and antibodies of the present invention have been found to produce surprisingly good results in fluorescence polarization assays for acetaminophen. Figure 1 shows the general structure of the acetaminophen that can be quantitatively determined in accordance with the present invention. The assay of the present invention provides a more rapid acetaminophen assay method than most prior art methods, because it requires no specimen treatment before analysis. The assay system accurately measures the quantity of acetaminophen in a sample, because the assay has minimal cross-reactivity to acetaminophen-like compounds.

In accordance with the analytical methods of the present invention, i.e. the methods of determining acetaminophen by a fluorescence immunoassay procedure using the tracer compounds and immunogens of the invention, a sample containing or suspected of containing acetaminophen is intermixed with a biologically acceptable salt of a tracer and an antibody specific to acetaminophen and the tracer. The antibody is produced using the immunogen as described above. The acetaminophen and tracer compete for limited antibody sites, resulting in the formation of complexes. By maintaining constant the concentration of tracer and antibody, the ratio of acetaminophen-antibody complex to tracer-antibody complex that is formed is directly proportional to the amount of acetaminophen in the sample. Therefore, upon exciting the mixture with linearly polarized light and measuring the polarization of the fluorescence emitted by a tracer and a tracer-antibody complex, one is able to quantitatively determine the amount of acetaminophen in the sample.

The results can be quantified in terms of net millipolarization units, span (in millipolarization units) and relative intensity. The measurement of millipolarization units indicates the maximum polarization when a maximum amount of the tracer is bound to the antibody in the absence of any acetaminophen. The higher the net millipolarization units, the better the binding of the tracer to the antibody.

The span is an indication of the difference between the net millipolarization and the minimum amount of tracer bound to the antibody. A larger span provides for a better numerical analysis of data. The intensity is a measure of the strength of the signal above background. Thus, a higher intensity will give a more accurate measurement. The intensity is determined at about 0.9 to 2.2 nanomolar for the preferred tracers of the invention, as the sum of the vertically polarized intensity plus twice the horizontally polarized intensity. The intensity can range from a signal from about three times to about thirty times the background noise, depending upon the concentration of the tracer and other assay variables. For the purposes of the present invention, an intensity aimed roughly at about eight to ten times that of background noise is preferred.

Table I shows the results obtained with various embodiments of the present invention, in terms of span, millipolarization units and intensity. In all instances, bovine serum albumin (BSA) was used as the protein carrier. As seen from the data in Table I, an assay produced from an immunogen made from the hapten of Figure 18 used in combination with the tracer of

Figure 17 provides excellent results. Accordingly, this combination is presently the most preferred form of the invention. In addition, the hapten/tracer combinations represented by the combinations of Figures 18 and 12, Figures 18 and 13, Figures 19 and 13, Figures 19 and 14 an Figures 20 and 13 also produced acceptable results and are alternative preferred combinations.

TABLE I

| Hapten used in Immunogen for Antibody | Tracer | Net Polarization* | Span* | Intensity** |
|---|---|---|---|---|
| FIG. 18 | FIG. 12 | 261 | 130 | 8.8 |
| FIG. 18 | FIG. 13 | 250 | 125 | 11.2 |
| FIG. 18 | FIG. 14 | 152 | NA | NA |
| FIG. 18 | FIG. 15 | 245 | 96 | 10.1 |
| FIG. 18 | FIG. 16 | 215 | 102 | 15.1 |
| FIG. 18 | FIG. 17 | 242 | 150 | 10.3 |
| FIG. 19 | FIG. 13 | 269 | 137 | 4.3 |
| FIG. 19 | FIG. 14 | 219 | 140 | 12.9 |
| FIG. 20 | FIG. 13 | 225 | 125 | 14.4 |
| FIG. 20 | FIG. 14 | 181 | 40 | 13.2 |

* In millipolarization units

** This is expressed as a ratio of net intensity to background noise

The pH at which the method of the present invention is practiced must be sufficient to allow the fluorescein moiety of the tracers to exist in their open form. The pH may range from about 3 to 12, more usually in the range of from about 5 to 10, most preferably from about 6 to 9. Various buffers may be used to achieve and maintain the pH during the assay procedure. Representative buffers include borate, phosphate, carbonate, tris, barbital and the like. The particular buffer employed is not critical to the present invention, but the tris and phosphate buffers are preferred. The cation portion of the buffer will generally determine the cation portion of the tracer salt in solution.

The preferred method of the improved assay of the present invention will now be discussed in detail. The assay is a "homogeneous assay," which means that the end polarization readings are taken from a solution in which bound tracer is not separated from unbound tracer. This is a distinct advantage over heterogeneous immunoassay procedures such as those where the bound tracer must be separated from the unbound tracer before a reading can be taken.

The reagents for the fluorescence polarization assay of the present invention comprise antibody specific for acetaminophen and tracer. Additionally, largely conventional solutions including a acetaminophen pretreatment solution, a dilution buffer, acetaminophen calibrators and acetaminophen controls are desirably prepared. Typical solutions of these reagents, some of which are described below, are commercially available in assay "kits" from Abbott Laboratories, Abbott Park, Illinois.

All percentages expressed herein are weight/-volume unless otherwise indicated. The tracer formulation presently preferred is 78 or 156 nanomolar tracer in: 0.1 molar tris buffer at pH 7.5; 0.2% sodium dodecyl sulfate; 0.1% sodium azide; and 0.01% bovine gamma-globulin. The antiserum formulation comprises sheep serum diluted with: 0.1 molar sodium phosphate buffer at pH 7.5; 0.1% sodium azide; 0.01% bovine gamma-globulin; and 2% ethylene glycol (volume/volume). The dilution buffer comprises: 0.1 molar sodium phosphate at pH 7.5; 0.1% sodium azide; and 0.01% bovine gamma-globulin. The pretreatment solution comprises: 0.1 molar tris buffer at pH 7.5; 0.1% sodium azide; 0.2% sodium dodecyl sulfate; and 0.01% bovine gamma-globulin Acetaminophen calibrators comprising acetaminophen in normal human serum at concentrations of 0.0, 10.0, 20.0, 50.0, 100.0 and 200.0 micrograms per milliliter with 0.1% sodium azide as a preservative are useful. Acetaminophen controls comprising acetaminophen in normal human serum are provided at concentrations of 15.0, 35.0 and 150.0 micrograms per milliliter with 0.1% sodium azide as a preservative are also useful.

The preferred procedure is especially designed to be used in conjunction with the Abbott TDx® Analyzer available from Abbott Laboratories, Irving, Texas. Fifty microliters of serum or plasma are required. The calibrators, controls, or unknown samples are pipetted directly into the sample well of the TDx® sample cartridge. One of the advantages of this procedure is that the sample does not require any special preparation. The assay procedure from this point is fully automated.

If a manual assay is being performed, then the sample is mixed with the pretreatment solution in

dilution buffer and a background reading is taken. The tracer is then mixed with the assay. The antibody is then finally mixed into the test solution. After incubation, a fluorescence polarization reading is taken.

The fluorescence polarization value of each calibrator, control or sample is determined and is printed on the output tape of an instrument such as the Abbott TDx® Analyzer. A standard curve is generated in the instrument by plotting the polarization of each calibrator versus its concentration using a nonlinear regression analysis. The concentration of each control or sample is read off the stored calibration curve and printed on the output tape.

With respect to the foregoing preferred procedure, it should be noted that the tracer, antibody, pretreatment solution, calibrators and controls should be stored between about 2 and about 8°C while the dilution buffer should be stored at ambient temperature. A standard curve and controls should be run every two weeks, with each calibrator and control run in duplicate. Controls should be run with each batch, and all samples can be run in duplicate.

It should be understood that the foregoing detailed description and the following Examples are intended to be illustrative, but not limiting, with respect to the scope of the present invention. Various modifications will become apparent to one skilled in the art, and thus it is intended that the scope of the invention be defined solely by the claims and legal equivalents thereof.

## EXAMPLES

Examples I through XX describe experiments that were performed in accordance with the concepts of the present invention. Examples I and II are directed

to preparation of an immunogen useful for producing antibody; Examples III through XIII are directed to the synthesis of precursors for immunogens and tracers; and Examples XIV through XX are directed to the preparation of tracers.

Example I: Preparation of an Immunogen from the hapten of Figure 18 by alkyl coupling

The hapten of Figure 18 (152.04 mg) was dissolved in about 1.0 ml of dimethylforamide. Distilled water (9.0 ml) was added to the solution, followed by 79.86 mg of BSA. The solution was titrated to a pH of 10.0 with 1.0 N KOH, and the pH was monitored during the reaction to insure its maintenance at this value. The product was purified on a G-25 Sephadex column using 0.01 M carbonate buffer at pH 9.0 as the eluant. The product was then dialyzed against ammonium hydroxide followed by saline.

Example II: Preparation of an Immunogen from the hapten of Figure 19 by active ester coupling

One hundred milligrams of the hapten of Figure 19 was combined with 77.9 mg of N-hydroxysuccinimide and 138.6 mg of dicyclohexyl-carbodiimide and dissolved in 1.0 ml of pyridine. After about 1-½ hrs., dicyclohexylurea formed, showing that the reaction had taken place. Approximately 100 mg of BSA was then dissolved in distilled water. The hapten solution was filtered and added dropwise into the BSA solution while mixing the latter. The reaction proceeded overnight at ambient temperature. The product was purified on a G-25 Sephadex column using a phosphate buffer as the eluent, and followed by dialysis against water.

Example III: 4-(Chloroacetamido)phenol

4-Aminophenol (2.2g) was suspended in 50 ml acetone, and chloroacetyl chloride (5.6g) in 10 ml

acetone was added dropwise to this suspension. The mixture was refluxed for 1 hour. After cooling to room temperature, 50 ml water was added. The acetone was removed *in vacuo* and water (50 ml) was added. The solution was cooled to about 10°C for 1 hour. The resulting solid product was filtered to yield 2.3g.

Example IV: 4-(Iodoacetamido)phenol

4-(2-Chloroacetylamino)phenol (1.86g) and sodium iodide (3.00g) were dissolved in 30 ml acetone and refluxed for 1 hour. The hot solution was filtered and washed with acetone. The combined filtrates were added to 50 ml water and extracted twice with 25 ml methylene chloride. The combined extracts were dried over sodium sulfate and sodium bisulfite to remove free iodide. After filtration, the solvent was removed *in vacuo*. A tan solid residue (2.0g) resulted.

Example V: 4-(4-Carboxybutyramido)phenol

4-Aminophenol (0.50g) and glutaric anhydride (0.64g) were dissolved in 5 ml acetic acid and stirred at room temperature for 3 hours. The product precipitated as a milky white solid that was filtered and washed with acetic acid. The solid was dried *in vacuo* to yield 1.0g; m.p., 206-212°C.

Example VI: 4-(3-Carboxypropionamido)phenol

4-Aminophenol (2.00g) and succinic anhydride (2.20g) were dissolved in 40 ml acetic acid and stirred at room temperature for 3.5 hrs. The product precipitated as a white solid that was filtered and washed with a minimum of acetic acid. The solid was dried *in vacuo* to yield 3.1g; m.p., 163-166°C.

Example VII: 4-[N'-(tert-Butoxycarbonyl)glycinamido]-phenol

4-(tert-Butoxycarbonyl)glycine (1.0g) and dicyclohexylcarbodiimide (0.6g) were dissolved in 5 ml dry pyridine and stirred at room temperature for 10 minutes. The solution became cloudy and thick. 4-Aminophenol (0.31g) was added and the mixture was stirred at room temperature for about 45 hours. The solid mass was filtered and the filtrate was reduced *in vacuo*. The residue was dissolved in methylene chloride and the solution was washed with 0.1N hydrochloric acid until most of the brown color was removed (at least 3 times). The methylene chloride layer was added to a silica gel column and eluted with the appropriate mixture of chloroform and ethyl acetate to yield 0.35g of product.

Example VIII: 4-Glycinamidophenol

4-[N'-(tert-Butoxycarbonyl)glycinamido]phenol (0.35g) was stirred at room temperature in 30 ml 50% (volume/volume) methylene chloride/trifluoroacetic acid for one hour. The solvent was removed *in vacuo* to yield 0.33g of product as the trifluoroacetic acid salt.

Example IX: 4-[N-(tert-Butoxycarbonyl)amino]butric acid

4-Aminobutric acid (0.5g) and sodium bicarbonate (0.41g) were dissolved in 10 ml 1:1 (volume/volume) dioxane/water. Di-t-butyldicarbonate (1.11 ml) was added and the reaction was stirred at room temperature for about 18 hrs. The dioxane was removed *in vacuo* and 10 ml 0.1N hydrochloric acid was added. The solution was extracted with methylene chloride and the methylene chloride was dried over sodium sulfate. The solvent was removed *in vacuo* to yield 0.77g of a colorless oil.

--

Example X: 4-{ N-(tert-Butoxycarbonyl)amino}butramido)-phenol

4-/N-(tert-Butoxycarbonyl)amino butric acid (0.77g) and 1-hydroxybenzotriazole (0.65g) were dissolved in 10 ml. dry pyridine. Dicyclohexylcarbodiimide (2.32g) was added and the mixture was stirred for about one hour at room temperature. 4-Aminophenol (0.46g) was added and the cherry red solution was stirred for about 18 hours. The reacton was filtered and the filtrate was reduced in vacuo. The residue was dissolved in methylene chloride. The methylene chloride was washed with dilute hydrochloric acid and then extracted with 10% sodium hydroxide. The sodium hydroxide layer was washed with methylene chloride until the methylene chloride was colorless. The water layer was acidified to about pH 1 with concentrated hydrochloric acid, causing a white solid to form. The water layer was extracted with methylene chloride several times. The methylene chloride was added to a silica gel column packed with methylene chloride and the product was eluted with the correct mixture of ethylacetate and methylene chloride. The product crystallized from methylene chloride to yield 0.90g; m.p., 138-140°C.

Example XI: 4-(Aminobutramido)phenol

4-(4-{ N-(tert-Butoxycarbonyl)amino}butramido]-phenol (0.80g) was dissolved in 16 ml 50% (volume/volume) trifluoroacetic acid/methylene chloride and the solution was stirred at room temperature for 1 hour. The solvent was removed in vacuo to yield the product as the trifluoroacetic acid salt.

Example XII: N-(tert-Butoxycarbonyl)glycine-1-(4-chlorophenyl)amide

N-(tert-Butoxycarbonyl)glycine (1.4g) and 1-hydroxybenzotriazole (2.2g) were dissolved in 30 ml

dry pyridine, and dicyclohexylcarbodiimide (3.3g) was added. The mixture was stirred at room temperature for 10 minutes, then 4-chloroaniline (1.0g) was added. The mixture was stirred for about 18 hours and then filtered. Water (20 ml) was added and the reaction was filtered again. The solvent was removed in vacuo. The residue was suspended in methylene chloride and filtered. The filtrate was reduced in vacuo to about half the volume and filtered again. The filtrate was added to a silica gel column packed with methylene chloride and the product was eluted with the correct ratio of ethyl acetate and methylene chloride to yield 0.34g.

Example XIII: 1-Chloro-4 glycinamidobenzene

N-(tert-Butoxycarbonyl)glycine-1-(4-chlorophenyl)amide (0.34g) was dissolved in 30 ml 50% (volume/volume) trifluoroacetic acid/methylene chloride and stirred at room temperature for 1 hour. The solvent was removed in vacuo. The residue was dissolved in methylene chloride and extracted with 1N hydrochloric acid. The water layer was basified with potassium carbonate and extracted with methylene chloride. The methylene chloride was dried over sodium sulfate and removed in vacuo to yield the product as a white solid.

Example XIV: N-[4-Chloro-6-(5-fluoresceinylamino)-1,3,5-triazin-2-yl]glycine-1-(4-hydroxyphenyl)amide

4-Glycinamidophenol (2mg) and 5-[(4,6-dichloro-1,3,5-triazin-2-yl)amino]fluorescein (6mg) were dissolved in 2 ml 1:1 (volume/volume) methanol/dimethylsulfoxide and stirred at room temperature for 30 minutes. The product was purified on silica gel preparative plates eluted with the correct ratio of methanol and chloroform. The appropriate band was

repurified on silica gel preparative plates eluted with the correct ratio of methanol and chloroform.

Example XV: 5-[(4-Chloro-6-methoxy-1,3,5-triazin-2-yl)amino]fluorescein

Aminofluorescein isomer I (3.82g) in 200 ml dry methanol was filtered and cooled to 0°C. 2,4-Dichlor-6-methoxy-1,3,5-triazine (1.80g, Aldrich Chemical, Milwaukee, Wisconsin) in 15 ml chloroform was added dropwise with stirring at 0°C. After one hour, 5 ml concentrated hydrochloric acid was added. After one additional hour, the product was twice filtered and washed with 50 ml chloroform.

Example XVI: N-[4-(5-Fluoresceinylamino)-6-methoxy-1,3,5-triazin-2-yl]glycine-1-(4-hydroxyphenyl)amide

4-Glycinamidophenol (29mg) and 2-chloro-4-(fluorescein-5-ylamino)-6-methoxy-1,3,5-triazine (100mg) were dissolved in 5 ml methanol and 0.5 ml triethylamine. The mixture was stirred and refluxed for 5 hours. After cooling to room temperature, the product was purified on silica gel preparative plates eluted with the correct ratio of chloroform and methanol. The appropriate band was repurified on silica gel preparative plates eluted with the correct ratio of chloroform and methanol.

Example XVII: 6-[(4-Hydroxyphenyl)amino]carbonyl-fluorescein

6-Carboxyfluorescein (10mg), N-hydroxy-succinimide (3mg) and dicyclohexylcarbodiimide (10mg) were dissolved in 1 ml dry pyridine and stirred at room temperature for 10 minutes. 4-Aminophenol (3mg) was added and the mixture was stirred for 25 hours. The product was purified on silica gel preparative plates

eluted with the correct ratio of methanol and chloroform. The appropriate band was repurified on silica gel preparative plates eluted with the correct ratio of methanol and ethyl ether.

Example XVIII: N-(Fluorescein-6-ylcarbonyl)glycine-1-(4-hydroxyphenyl)amide

6-Carboxyfluorescein (30 mg, Calbiochem) and N, N'-dicyclohexylcarbodiimide (57 mg) were dissolved in 2 ml dry pyridine with stirring, then 1-hydroxybenzotriazole (11 mg) was added. After 30 minutes, 4-glycinamidophenol (12.6 mg) was added and stirred at room temperature for about 17 hours. The product was purified on silica gel preparative plates eluted with the correct ratio of chloroform and methanol.

Example XIX: N-(Fluorescein-5-ylcarboxyl)glycine-1-(4-hydroxyphenyl)amide

5-Carboxyfluorescein (30 mg, Calbiochem) and N, N'-dicyclohexylcarbodiimide (50 mg) were dissolved in 2 ml dry pyridine with stirring, then 1-hydroxybenzotriazole (11 mg) was added. After 30 minutes, 4-glycinamidophenol (12.6 mg) was added and stirred at room temperature for about 17 hours. The product was purified on silica gel preparative plates eluted with the correct ratio of chloroform and methanol.

Example XX: N-(Fluorescein-5-ylamino)-N'-(4-hydroxyphenyl)succinamide

4-(3-Carboxypropionamido)phenol (0.20 g), 1-hydroxybenzotriazole (0.15 g) and dicyclohexylcarbodiimide (0.22 g) were dissolved in dry pyridine at 0°C and stirred for 15 minutes. Aminofluorescein isomer I (0.34 g) was added and the reaction was stirred for 8 days at room temperature. The product

was purified on silica gel preparative plates eluted with the correct ratio of chloroform and methanol. The appropriate band was repurified on silica gel preparative plates eluted with the correct ratio of ethyl ether.

CLAIMS

1. A compound comprising the structure:

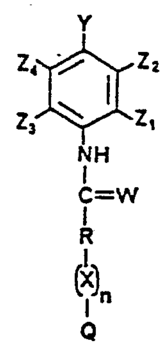

wherein

Q is a poly(amino acid), a poly(amino acid) derivative, fluorescein, or a fluorescein derivative;

X is NH or CO;

n is 0, 1 or 2;

R is a linking group including up to 2 heteroatoms when Q is a poly(amino acid) or a poly(amino acid) derivative and up to 4 heteroatoms having a total of from 0 to 8 carbon atoms and heteroatoms;

W is O or S when Q is fluorescein or a fluorescein derivative, and W is O when Q is a poly(amino acid) derivative;

Y is OH, $NH_2$, $CH_3$, F, Cl, Br or H when Q is fluorescein or a fluorescein derivative, and Y is OH, $NH_2$, $CH_3$, F or Cl when Q is a poly(amino acid) or a poly(amino acid) derivative; and

$Z_1$, $Z_2$, $Z_3$ and $Z_4$ are each independently H or F when Q is fluorescein or a fluorescein derivative, and are each H when Q is a poly(amino acid) or a poly(amino acid) derivative.

2. The compound of Claim 1 wherein Q is bovine serum albumin.

3. The compound of Claim 1 wherein Q is an amino, an amido, an amidino, a urea, a thiourea, a carbamate, a thiocarbamate, a triazinylamino, or a (carboxyamino)-sulfonamido derivative of fluorescein.

4. A method for making an immunogen comprising the step of coupling a precursor of the formula

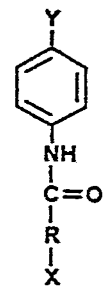

where:

X is $NH_2$, COOH, CN, CHO, Br, I or OH;

R is a linking group including up to 2 heteroatoms and having a total of from 0 to 8 carbon atoms and heteroatoms when X is COOH and a total of from 1 to 8 carbon atoms and heteroatoms when X is $NH_2$, CN, CHO, Br, I or OH; and

Y is OH, $CH_3$, F, Cl or $NH_2$, or, in cases where X is CHO, Br or I, a protected derivative of $NH_2$;

with a poly(amino acid) or a derivative of a poly(amino acid).

5. The method of Claim 4 wherein when X is $NH_2$ or $CO_2H$, the precursor is coupled by reacting it with the poly(amino acid) by addition of N,N'-dicyclohexyl-carbodiimide, 1-ethyl-3-(3-dimethyl-aminopropyl(carbo-diimide or 1-cyclohexyl-3-(2-morpholinoethyl)-carbodiimide metho-p-toluenesulfonate.

6. A method for making a tracer comprising the step of coupling a precursor of the formula

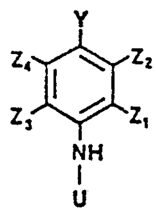

where:

U is H, or the structure $-\overset{\overset{W}{\|}}{C}-R-X$, where: W is O or S; R is a linking group including up to 4 heteroatoms and having a total of from 0 to 8 carbon atoms and heteroatoms when X is COOH, and having a total of from 1 to 8 carbon atoms and heteroatoms when X is $NH_2$, CN or OH; and X is $NH_2$, COOH, CN or OH;

Y is OH, $NH_2$, $CH_3$, F, Cl, Br or H; and

$Z_1$, $Z_2$, $Z_3$ and $Z_4$ are each independently H or F;

with fluorescein or a derivative of fluorescein.

7. The method of Claim 6 wherein when U is H or X is $NH_2$, the precursor is coupled by the steps of:

(a) preparing an active ester of carboxyfluorescein;

(b) reacting the active ester with the precursor.

8. The method of Claim 6 wherein when X is $CO_2H$, the precursor is coupled by the steps of:

(a) in cases where Y is $NH_2$, protecting the $NH_2$ group;

(b) preparing an active ester of the precursor;

(c) reacting the active ester with aminofluorescein.

9. A process for measuring concentration of acetaminophen which comprises the steps of:

(a) contacting a sample with a acetaminophen antiserum and with a compound according to Claim 1 capable of producing a detectable fluorescence polarization response to the presence of the acetaminophen antiserum;

(b) passing plane polarized light through the resulting solution from step (a) to obtain a fluorescence polarization response; and

(c) detecting the fluorescence polarization response of the solution of step (b) as a measure of the amount of acetaminophen in the sample.

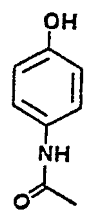

Fig. 1

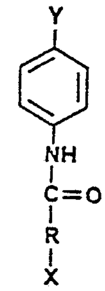

Fig. 2

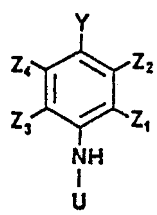

Fig. 3

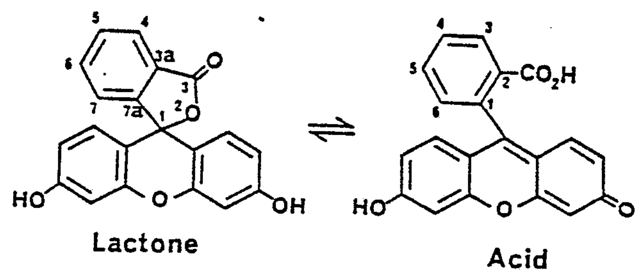


Fig. 4

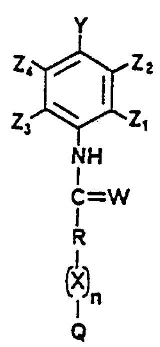

Fig. 5

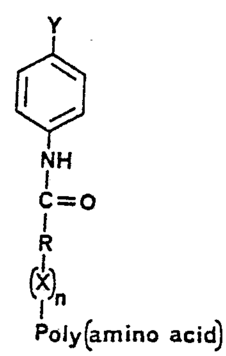

Fig. 6

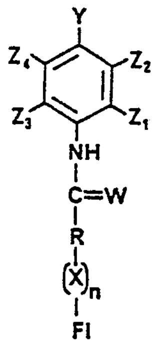

Fig. 7

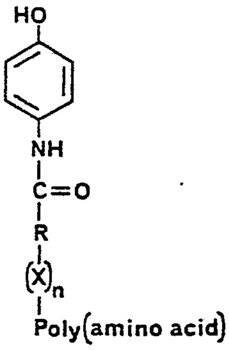

Fig. 8

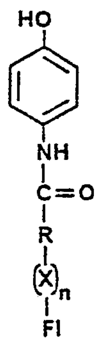

Fig. 9

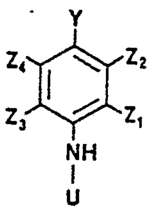

Fig. 10

FIG. 11

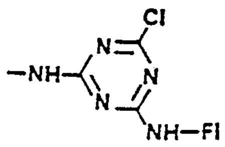

FIG. 11-1

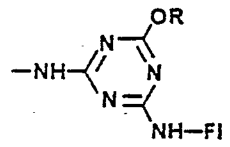

FIG. 11-2

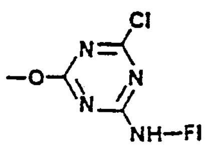

FIG. 11-3

-NH-CO-Fl

FIG. 11-4

-CO-NH-Fl

FIG. 11-5

-CNH-NH-Fl

FIG. 11-6

-NH-CO-NH-Fl

FIG. 11-7

-NH-CS-NH-Fl

FIG. 11-8

-O-CO-NH-Fl

FIG. 11-9

-O-CS-NH-Fl

FIG. 11-10

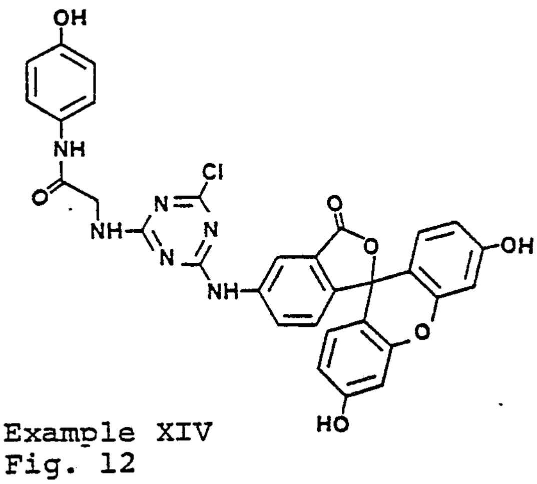

Example XIV
Fig. 12

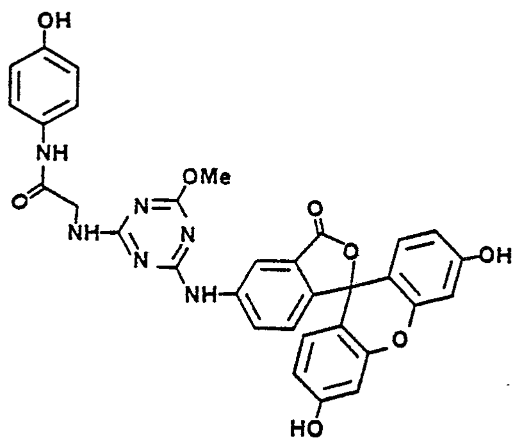

Example XVI
Fig. 13

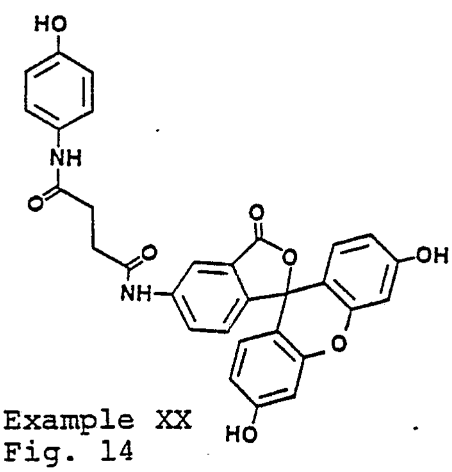

Example XX
Fig. 14

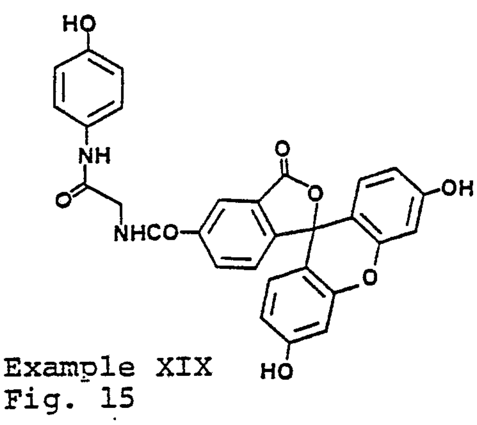

Example XIX
Fig. 15

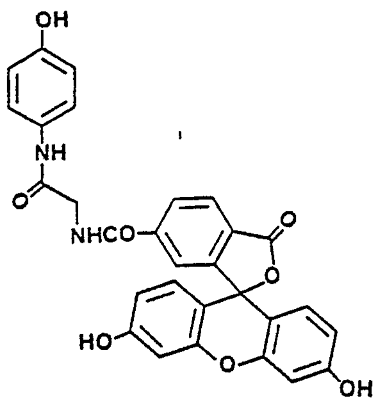

Example XVIII
Fig. 16

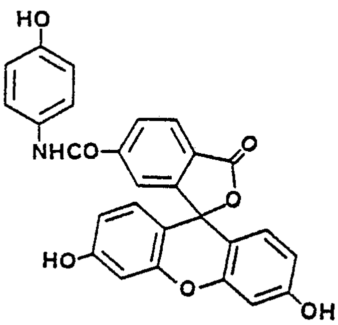

Example XVII
Fig. 17

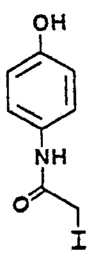

Example IV
Fig. 18

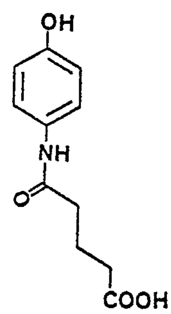

Example V
Fig. 19

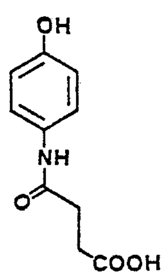

Example VI
Fig. 20